# EUROPÄISCHE PATENTSCHRIFT

(11) **EP 2 173 295 B1**
(45) Veröffentlichungstag und Bekanntmachung des Hinweises auf die Patenterteilung: **19.12.2018**
(21) Anmeldenummer: 08784747.1
(22) Anmeldetag: 12.07.2008
(51) Int. Cl.: A61F 13/00, A61F 13/36

(54) **MEDIZINISCHE KOMPRESSE I**
MEDICAL COMPRESS I
COMPRESSE MÉDICALE

(30) Priorität: 01.08.2007 DE 102007036083
(43) Veröffentlichungstag der Anmeldung: 14.04.2010
(73) Patentinhaber: Paul Hartmann AG, 89522 Heidenheim (DE)
(72) Erfinder: HOFSTETTER, Jürgen, 89522 Heidenheim (DE); SCHMID, Horst, 89567 Sontheim (DE); HALBAUER, Rainer, 73569 Obergröningen (DE)
(86) Internationale Anmeldenummer: PCT/EP2008/005718
(87) Internationale Veröffentlichungsnummer: WO 2009/015758

(56) Entgegenhaltungen:
- BE-A- 505 633
- GB-A- 782 861
- GB-A- 1 410 810

## Beschreibung

Die vorliegende Erfindung betrifft medizinische Kompressen nach Ansprüche 1-8 aus einem textilen Flachbahnmaterial, insbesondere Mullkompressen, in einer anwenderfreundlichen Form. Weiterhin betrifft die Erfindung ein Verfahren zur Herstellung dieser Kompressen. Medizinische Kompressen sind seit langer Zeit zur Behandlung von akuten Wunden in der Notfallmedizin oder zur Verwendung bei chirurgischen Eingriffen bekannt. Im Wesentlichen werden diese Kompressen hinsichtlich der eingesetzten Materialien unterschieden und mit Hilfe dieser Unterscheidung in Mullkompressen und Vliesstoffkompressen eingeteilt. Dabei werden Mullkompressen üblicherweise aus einem Baumwollgewebe hergestellt, das je nach Fadendichte eine grobe oder eine feine Gitterstruktur aufweist. Die Anforderungen an den Mull zur Bildung von Kompressen sind mit der DIN EN 14079 festgelegt.

Da es sich bei den Mullkompressen um eine Gitterstruktur handelt, die den Nachteil aufweist, dass endständige Fäden sich lösen können, sind vielfach Lösungen unterbreitet worden, dieses Ablösen zu verhindern. So wird beispielsweise mit der DE 2261889 eine Kompresse beschrieben, die mindestens einen vermaschten Streifen aufweist. Durch die Vermaschung der Fäden werden zwei gegenüberliegende Ränder eines Bandmaterials gebildet, aus denen keine Fäden ausgelöst werden können. Die Kompresse wird letztendlich aus einem Teilabschnitt dieses Bandmaterials gebildet, wobei die Schnittkanten dieses Abschnittes eingeschlagen werden. Darüber hinaus wird mit der DE 9014500 eine Mullkompresse vorgeschlagen, die in unmittelbarer Nähe zu einer Schnittkante thermoplastische Fäden, Bänder, Streifen oder Vliesstreifen aufweist. Diese zusätzlichen Materialien werden mit dem Mull verschweißt, versiegelt oder verklebt. Als im Markt bekannte Mullkompressen haben sich beispielsweise ES-Kompressen etabliert. Diesen vorgeschlagenen Lösungen oder vorhandenen Produkten ist gemeinsam, dass sie als zu aufwendig und/oder zu kostenintensiv in der Fertigung angesehen werden.

Aufgabe der vorliegenden Erfindung ist es, textile Kompressen bereitzustellen, die handhabungssicher und kostengünstig in der Fertigung sind. Des Weiteren soll eine Mehrzahl solcher Kompressen leicht stapelbar sein, wobei die Kompressen ein möglichst geringes Packvolumen einnehmen sollen. Zudem soll ein Verfahren zur Herstellung von medizinischen Kompressen bereitgestellt werden.

Gelöst werden diese Aufgaben von einer medizinischen Kompresse gemäß Anspruch 1. Demnach umfasst eine erfindungsgemäße medizinische Kompresse mindestens 8 Lagen eines textilen Flachbahnmaterials, wobei jede Lage über mindestens eine Faltkante mit einer weiteren Lage verbunden ist und wobei mindestens eine erste Faltkante und eine zweite Faltkante senkrecht zueinander stehen. Die Kompresse ist derart gefaltet, dass die Kompresse mindestens zwei Faltkanten als erste Stoßkanten umfasst, wobei jede dieser ersten Stoßkanten direkt benachbarte Lagen oder Teilabschnitte der direkt benachbarten Lagen verbindet. Insbesondere weist die medizinische Kompresse zwei Faltkanten als erste Stoßkanten und zwei Schnittkanten als zweite Stoßkanten auf.

Im Zusammenhang mit der vorliegenden Erfindung soll unter einer Faltkante eine Kante oder ein Teilabschnitt der Kante verstanden sein, die durch das vollständige oder teilweise Übereinanderlegen, Übereinanderschlagen oder Einschlagen von zwei Untereinheiten des Materialabschnitts gebildet wird, wobei die beiden Untereinheiten des Materialabschnitts mittels der Faltkante verbunden sind. Hierbei können die beiden Untereinheiten des Materialabschnitts nach dem Übereinanderlegen, Übereinanderschlagen oder Einschlagen direkt benachbart sein, das heißt, dass die beiden Untereinheiten des Materialabschnitts im direkten Kontakt stehen, oder durch weitere Lagen beabstandet sein.

Eine Schnittkante eines Materialabschnitts des Flachbahnmaterials ist demgegenüber eine Kante, die durch das Trennen von einem ersten Materialabschnitt aus einem den ersten Materialabschnitt umfassenden größeren Materialabschnitt gebildet wird, wobei jeweils eine Schnittkante dem ersten Materialabschnitt und eine Schnittkante dem verbleibenden Materialabschnitt zugeordnet werden kann. Hierbei können alle heute bekannten Trenntechniken, wie das Schneiden mittels Messer oder Scheren, Laserstrahlen, Wasserstrahlen und andere Techniken eingesetzt werden.

Weiterhin soll unter einer Stoßkante eine Schnitt- oder Faltkante sowie Teilabschnitte dieser Schnitt- oder Faltkanten verstanden sein, die durch das Anlegen dieser Schnitt- oder Faltkante oder dieser Teilabschnitte der Schnitt- oder Faltkanten gegen eine weitere Schnittkante oder eine weitere Faltkante bzw. Teilabschnitte der weiteren Schnittkante oder weiteren Faltkante gebildet wird, wobei die beteiligten Schnitt- oder Faltkanten bzw. deren Teilabschnitte in einer Ebene liegen. Idealer Weise liegen die Stoßkanten einer erfindungsgemäßen Kompresse direkt an- oder gegeneinander. Gemäß der vorliegenden Erfindung werden jedoch auch solche Kanten als Stoßkanten bezeichnet, deren beteiligte Schnitt- oder Faltkanten geringfügig, das heißt höchstens 15 %, von einander beabstandet sind und/ oder bis zu 15 % überlappen, wobei der jeweilige Wert des Abstands oder der Überlappung auf die Länge einer Randkante der Kompresse im endgefalteten Zustand bezogen wird und als Bemessungsgrundlage diejenige Randkante der Kompresse verwendet wird, die dem Betrag nach den größten Wert aufweist.

Des Weiteren soll im Zusammenhang mit der vorliegenden Erfindung (falls nicht anders angezeigt) unter einer Randkante eine Außenkante der endgefertigten Kompresse verstanden sein.

Durch die Ausbildung von mindestens zwei Faltkanten als erste Stoßkanten kann eine Kompresse bereitgestellt werden, die keine freien Schnittkanten aufweist und zudem eine Materialersparnis und damit eine Kostenreduktion in der Fertigung ermöglicht. Durch die Ausbildung von Stoßkanten wird weiterhin verhindert, dass sich Fäden vom Schnittrand lösen und bei der bestimmungsgemäßen Verwendung in eine Wunde gelangen. Zudem weist eine erfindungsgemäße Kompresse den Vorteil auf, dass auch beim einmaligen Entfalten der fertigen insbesondere mindestens 8-lagigen Kompresse keine Schnittkanten freigelegt werden. Diese Kompresse ist besonders handhabungssicher und darüber hinaus anwenderfreundlich, da der Anwender selbst festlegen kann, ob er beispielsweise eine 8-lagige Kompresse als eine 4-lagige oder als eine 8-lagige Kompresse nutzen möchte. In beiden Fällen liegen keine Schnittkanten frei.

Als vorteilhaft hat sich weiterhin herausgestellt, wenn die Kompresse derart gefaltet ist, dass die äußeren, die Auflageflächen der Kompresse bildende Lagen die Form eines Rechtecks oder eines Quadrates aufweisen. Insbesondere ist die medizinische Kompresse derart gefaltet, dass die äußeren, die Auflageflächen der Kompresse bildenden Lagen vollständig durch jeweils einen zusammenhängenden Bereich des Flachbahnmaterials gebildet sind, also keine Stoßkanten aufweisen, wobei die Auflageflächen rechteckig oder quadratisch sind. Die Kompresse ist derart gefaltet, dass alle Randkanten der endgefertigten Kompresse aus Faltkanten gebildet sind.

Diese Auflageflächen können verwirklicht werden, indem eine erfindungsgemäße Kompresse aus einem rechteckigen Materialabschnitt des Flachbahnmaterials mit zwei sich gegenüber liegenden parallelen Schnittkanten A und zwei sich gegenüber liegenden parallelen Schnittkanten B gefertigt wird, wobei die Kantenlänge a der Schnittkanten A größer oder gleich der Kantenlänge b der Schnittkanten B ist, und jede Schnitt-, Falt-, Stoß- und/ oder Randkante der Kompresse parallel oder senkrecht zu einer weiteren Schnitt-, Falt-, Stoß- und/ oder Randkante der Kompresse liegt und wobei mindestens eine Schnitt-, Falt- oder Stoßkante der Kompresse senkrecht zu einer weiteren Schnitt-, Falt- oder Stoßkante der Kompresse liegt. Insbesondere ist die Kantenlänge a der Schnittkanten A größer als die Kantenlänge b der Schnittkanten B. Weiterhin bevorzugt bilden hierbei Faltkanten, die parallel zu den Schnittkanten B des Materialabschnitts gebildet sind, die ersten Stoßkanten, wobei ganz besonders bevorzugt die Schnittkanten A die zweiten Stoßkanten bilden. Damit ist eine erfindungsgemäße Kompresse insbesondere aus einem rechteckigen Materialabschnitt mit den Schnittkanten A und B des textilen Flachbahnmaterials gebildet, wobei die Kantenlänge a der Schnittkanten A größer ist als die Kantenlänge b der Schnittkanten B. Damit weist eine erfindungsgemäße Kompresse vorzugsweise auch rechteckige oder quadratische Auflageflächen mit den Randkanten C und D auf, wobei die Kantenlänge c der Randkanten C größer oder gleich der Kantenlänge d der Randkanten D ist, und ist vorzugsweise derart gefaltet, dass die ersten Stoßkanten parallel zu den ersten Faltkanten liegen und insbesondere parallel zu den Randkanten D liegen. Insbesondere weist eine erfindungsgemäße Kompresse quadratische Auflageflächen F mit Randkanten D mit der Kantenlänge d auf. Es kann jedoch auch vorgesehen sein, dass eine erfindungsgemäße Kompresse rechteckige Auflageflächen mit den Randkanten C und D aufweist, wobei die Kantenlänge c der Randkante C größer ist als die Kantenlänge d der Randkante D, und parallel zu der Rankante D liegende erste Faltkanten als erste Stoßkanten aufweist. In diesem Fall liegen die Schnittkanten A des rechteckigen Materialabschnitts parallel zu den Randkanten C und die Schnittkanten B des rechteckigen Materialabschnitts parallel zu den Randkanten D. Anzumerken ist an dieser Stelle, dass erfindungsgemäße Kompressen sowohl maschinell als auch von Hand gefertigt werden können.

Grundsätzlich kann eine Kompresse aus einem rechteckigen Materialabschnitt eines Flachbahnmaterials mit zwei sich gegenüber liegenden parallelen Schnittkanten A und zwei sich gegenüber liegenden parallelen Schnittkanten B gefertigt werden, wobei die Kantenlänge a der Schnittkanten A größer ist als die Kantenlänge b der Schnittkanten B. Jedoch kann bei der Ausbildung von ersten Stoßkanten aus Faltkanten, die parallel zu den Schnittkanten B gebildet werden, insbesondere aus ersten Faltkanten gebildete Stoßkanten, im Vergleich zu Stoßkanten, die aus Faltkanten parallel zu Schnittkanten A gebildet werden, also aus zweiten Faltkanten gebildete Stoßkanten, eine erhebliche Materialersparnis verwirklicht werden. Diese Materialersparnis beläuft beispielsweise für eine quadratische, 8-lagige Kompresse bei gleichem Material in Abhängigkeit von der Auflagefläche und der Breite der zuerst umgeschlagenen Teilbereiche auf 5 - 15 %.

Gemäß weiteren Ausführungen weist eine erfindungsgemäße Kompresse mindestens 8 und höchstens 16 Lagen auf. Insbesondere handelt es sich bei einer erfindungsgemäßen Kompresse um eine Kompresse, die im Querschnitt betrachtet 8-lagige und 10- oder 12-lagige Bereiche aufweist. Hierunter ist zu verstehen, dass die Kompresse im Querschnitt betrachtet nicht über den gesamten Bereich ihrer Quer- oder Längserstreckung einen homogenen Lagenaufbau aufweist, sondern dass die Kompresse in einem ersten Teilbereich 10- oder 12-lagig und in mindestens einem weiteren Teilbereich 8-lagig ist. Ganz besonders bevorzug weist die Kompresse einen ersten Randbereich auf, der 8-lagig ist, und einen mittleren Bereich der 10-lagig ist. Es kann jedoch auch vorgesehen sein, das die Kompresse einen homogenen Lagenaufbau von entweder 10 Lagen oder 12 Lagen oder 16 Lagen aufweist.

Insbesondere bilden hierbei zu den ersten Faltkanten parallele Faltkanten die Randkanten C der endgefalteten Kompresse und zu den zweiten Faltkanten parallele Faltkanten die Randkante D der endgefalteten Kompresse, wobei weiterhin bevorzugt die erste Faltkante die beiden äußeren, die Auflageflächen der Kompresse bildenden Materialabschnitte miteinander verbindet und die zweite Randkante senkrecht hierzu steht. Zu dem hat sich herausgestellt, dass eine erfindungsgemäße Kompresse, deren Randkanten ausschließlich aus Faltkanten gebildet werden, besonders handhabungssicher ist. Diese Anordnung der Lagen weist den Vorteil auf, dass jede weitere Lage der Kompresse zwischen den beiden äußeren Lagen angeordnet ist und somit eine vom Anwender leicht greifbare Kompresse bereitgestellt werden kann. So kann selbst wenn die erfindungsgemäßen Kompressen in einem Stapel angeordnet sind, eine Kompresse ergriffen werden ohne unbeabsichtigt eine weitere Lage einer benachbarten Kompresse zu ergreifen.

In einer weiteren Ausführung der Erfindung kann auch vorgesehen sein, dass die Kompresse zwei weitere Schnitt- und/ oder Faltkanten als zweite Stoßkanten umfasst. Insbesondere liegen diese zweiten Stoßkanten parallel zu der zweiten Faltkante. Weiterhin bevorzugt liegen die zweiten Stoßkanten senkrecht zu den ersten Stoßkanten. Somit kann sichergestellt werden, dass alle Schnittkanten im Inneren der Kompresse angeordnet werden.

Gemäß der vorliegenden Erfindung bilden die ersten Stoßkanten bildenden Faltkanten keine Randkanten der Kompresse. Die ersten Stoßkanten liegen somit immer zwischen zwei insbesondere parallel liegenden Randkanten. Gemäß einer besonders bevorzugten Ausführung der vorliegenden Erfindung ist dabei vorgesehen, dass die Kompresse erste Stoßkanten aufweist, die in jedem Punkt einen Abstand von einer ersten Randkante von mindestens 25 % und höchstens 75 % des Betrags der Länge einer zweiten, senkrecht zur ersten Randkante liegenden Randkante aufweisen. Besonders bevorzugt weisen diese Kompressen erste Stoßkanten auf, die in jedem Punkt einen Abstand von einer ersten Randkante von mindestens 40 % und höchstens 60 % des Betrags der Länge einer zweiten, senkrecht zur ersten Randkante liegenden Randkante aufweisen. Ganz besonders bevorzugt weisen diese Kompressen erste Stoßkanten auf, die in jedem Punkt einen Abstand von einer ersten Randkante von mindestens 45 % und höchstens 55 % des Betrags der Länge einer zweiten, senkrecht zur ersten Randkante liegenden Randkante aufweisen.

Somit wird mit einer bevorzugten Ausführungsform der vorliegenden Erfindung eine Kompresse bereitgestellt, die derart gefaltet ist, dass die eingeschlagene Schnittkanten nicht wie bei bekannten ES-Kompressen am Rand übereinander liegen, sondern in der Mitte der Kompresse nebeneinander liegen. Dies ergibt den zusätzlichen Nutzen, dass dort, wo es sinnvoll ist, zwei Lagen zusätzlich vorhanden sind.

Zudem wird eine Kompresse bereitgestellt, die flacher ist und damit weniger Lagerraum einnimmt. Im weiteren Vergleich zu einer bekannten 8-lagigen ES-Kompresse weist eine erfindungsgemäße 8-lagige Kompresse an ihrer dicksten Stelle nur 10 Lagen auf, wogegen die bekannte ES-Kompresse 16 Lagen aufweist. Durch die erfindungsgemäße Faltung kann nun eine Kompresse bereitgestellt werden, die viel leichter stapelbar ist.

Zur Herstellung einer erfindungsgemäßen Kompresse kann als Flachbahnmaterial jedwede textile Flachbahnmaterialien eingesetzt werden, die verschieden von nichtgewebten Flachbahnmaterialien, den so genannten Nonwoven oder Vliesstoffen sind. Die vorliegende Erfindung betrifft keine Vliesstoffkompressen. Als textile Flachbahnmaterialien können erfindungsgemäß insbesondere Gewebe, Gewirke oder Gestricke zum Einsatz gebracht werden. Ganz besonders bevorzugt werden Gewebe und insbesondere Gewebe mit einer Leinwandbindung zum Einsatz gebracht.

Die textilen Flachbahnmaterialien können weiterhin bevorzugt aus einem Garn- oder Fasermaterial hergestellt sein, das Fasern oder Filamente natürlichen Ursprungs und/ oder synthetische Fasern umfasst. Als Fasern natürlichen Ursprungs umfasst eine erfindungsgemäße Kompresse insbesondere Fasern aus Baumwolle, Hanf, Flachs oder Leinen. Falls das Flachbahnmaterial Garn- oder Fasermaterial umfasst, das synthetische Fasern umfasst, so können hierzu Fasern oder Filamente aus Viskose, Polyester, Celluloseacetat, Carboxymethylcellulose, Hydroxyethylcellulose verwendet werden. Ganz besonders bevorzugt umfasst das textile Flachbahnmaterial ein Garn- oder Fasermaterial aus Baumwolle und/ oder Viskose, das den Anforderungen der DIN EN 14079 genügt.

Diese Materialien, insbesondere Mull, sind im Vergleich zu bekannten nicht-textilen oder nicht-gewebten Flachbahnmaterialien wie Nonwoven oder Vliesstoffe nicht in einem stufenlosen, kontinuierlichen Verfahren zu verarbeiten. Aus diesem Grund müssen erfindungsgemäße Kompressen aus einem diskreten Materialabschnitt gefertigt werden.

Eine besonders bevorzugte Ausführung einer erfindungsgemäßen medizinischen Kompresse ist eine Mullkompresse. Diese Mullkompresse umfasst mindestens 8 Lagen Mull gemäß DIN EN 14079, wobei jede Lage über mindestens eine Faltkante mit einer weiteren Lage verbunden ist und wobei mindestens eine erste Faltkante und eine zweite Faltkante senkrecht zueinander stehen. Die Mullkompresse ist derart gefaltet, dass die Kompresse mindestens zwei Faltkanten als erste Stoßkanten umfasst, wobei jede dieser ersten Stoßkanten direkt benachbarte Lagen verbindet. Insbesondere weist die medizinische Kompresse zwei Faltkanten als erste Stoßkanten und zwei Schnittkanten als zweite Stoßkanten auf. Des Weiteren kann diese Mullkompresse alle weiteren Merkmale der zuvor beschriebenen Art einzeln oder in Kombination umfassen.

Gemäß einem weiterführenden Gedanken der vorliegenden Erfindung ist auch ein Kompressenstapel umfassend eine Mehrzahl von erfindungsgemäßen medizinischen Kompressen Gegenstand der vorliegenden Erfindung. Dieser Stapel umfasst eine Mehrzahl an Kompressen der zuvor beschriebenen Art. Insbesondere kann dieser Stapel eine Mehrzahl an gleichen Kompressen umfassen, wobei jede Kompresse einzelne Merkmale oder Kombinationen von Merkmalen der zuvor beschriebenen Kompressen aufweist.

Demnach ist insbesondere auch ein Kompressenstapel umfassend eine Mehrzahl von medizinischen Kompressen umfassend mindestens 8 Lagen eines textilen Flachbahnmaterials, insbesondere eine Mehrzahl an Mullkompressen umfassend 8 Lagen Mull gemäß DIN EN 14079, Gegenstand der vorliegenden Erfindung. Jede dieser mindestens 8 Lagen jeder einzelnen Kompresse ist über mindestens eine Faltkante mit einer weiteren Lage dieser Kompresse verbunden, wobei mindestens eine erste und eine zweite Faltkante senkrecht zueinander stehen. Jede dieser Kompressen ist derart gefaltet, dass sie mindestens zwei Faltkanten als erste Stoßkanten umfasst, wobei jede dieser ersten Stoßkanten direkt benachbarte Lagen oder Teilabschnitte der direkt benachbarten Lagen verbindet.

Durch die Anordnung der Faltkanten als Stoßkanten kann ein Kompressenstapel bereitgestellt werden, der im Vergleich zu im Markt befindlichen Kompressen in sich stabiler ist und weniger Platz einnimmt. Hierdurch kann insbesondere Verpackungsmaterial eingespart werden. Werden beispielsweise im Markt befindliche ES-Kompressen gestapelt, so weist die Verpackung mit einem Kompressenstapel von 100 Kompressen eine Höhe von 155 mm (Außenmaß der Verpackung) auf. Werden dagegen erfindungsgemäße Kompressen gestapelt (100 Stück) unter denselben Bedingungen verpackt, so ist ein Außenmaß von 130 mm zu verzeichnen (dieselben Messbedingungen). Somit kann insbesondere Verpackungsmaterial und Lagerraum eingespart werden.

Weiterhin bevorzugt weist ein erfindungsgemäßer Stapel Kompressen mit rechteckigen oder quadratischen Auflageflächen auf, wobei jede Kompresse zwei sich gegenüberliegende Randkanten C mit einer Kantenlänge c und zwei sich gegenüberliegende Randkanten D mit einer Kantenlänge d aufweist, und wobei die Kantenlänge c größer oder gleich der Kantenlänge d ist. Insbesondere weist dieser Stapel eine Mehrzahl an Kompressen mit quadratischen Auflageflächen auf.

In einer weiteren Ausführung der vorliegenden Erfindung weist ein erfindungsgemäßer Kompressenstapel eine Mehrzahl an Kompressen auf, deren erste Stoßkanten in jedem ihrer Punkte einen Abstand von einer ersten Randkante von mindestens 25 % und höchstens 75 % des Betrags der Länge einer zweiten, senkrecht zur ersten Randkante liegenden Randkante aufweisen. Besonders bevorzugt weisen diese Kompressen erste Stoßkanten auf, die in jedem Punkt einen Abstand von einer ersten Randkante von mindestens 40 % und höchstens 60 % des Betrags der Länge einer zweiten, senkrecht zur ersten Randkante liegenden Randkante aufweisen. Ganz besonders bevorzugt weisen diese Kompressen erste Stoßkanten auf, die in jedem Punkt einen Abstand von einer ersten Randkante von mindestens 45 % und höchstens 55 % des Betrags der Länge einer zweiten, senkrecht zur ersten Randkante liegenden Randkante aufweisen.

Diese Kompressen sind derart übereinander gestapelt, dass jeweils eine erste Auflagefläche einer ersten Kompresse deckungsgleich mit einer ersten Auflagefläche einer zweiten oder weiteren Kompresse aufeinander liegt. Hierbei kann weiterhin bevorzugt sein, dass jede erste Faltkante einer Kompresse, die die beiden äußeren, die Auflageflächen der Kompresse bildenden Lagen des textilen Flachbahnmaterials miteinander verbindet, deckungsgleich übereinander liegen.

In Fortführung der Erfindung ist auch ein Verfahren zur Herstellung einer medizinischen Kompresse mit mindestens 8 Lagen eines textilen Flachbahnmaterials, wobei jede Lage über mindestens eine Faltkante mit einer weiteren Lage verbunden ist und mindestens eine erste und eine zweite Faltkante senkrecht zueinander stehen, Gegenstand der vorliegenden Erfindung. Insbesondere soll ein Verfahren zur Herstellung einer Kompresse der zuvor beschriebenen Art bereitgestellt werden. Das Verfahren umfasst die Verfahrensschritte
a) Bereitstellen eines rechteckigen Materialabschnitts des Flachbahnmaterials mit zwei sich gegenüberliegenden ersten Schnittkanten A und zwei sich gegenüberliegenden zweiten Schnittkanten B, wobei die Kantenlänge a der Schnittkante A größer oder gleich ist der Kantenlänge b der Schnittkante B,
b) Einschlagen der zweiten Schnittkanten B des rechteckigen Materialabschnitts zur Ausbildung von zwei ersten Faltkanten
c) Einschlagen der unter b) ausgebildeten Faltkanten zur Ausbildung von zwei ersten Stoßkanten der Kompresse,
d) weiteres Einschlagen von Faltkanten zur Ausbildung weiterer Falt- oder Stoßkanten der Kompresse.

Insbesondere umfasst das Verfahren den Verfahrensschritt a) Bereitstellen eines rechteckigen Materialabschnitts des Flachbahnmaterials mit zwei sich gegenüberliegenden ersten Schnittkanten A und zwei sich gegenüberliegenden zweiten Schnittkanten B, wobei die Kantenlänge a der Schnittkante A größer ist als die Kantenlänge b der Schnittkante B.

Weiterhin bevorzugt erfolgt innerhalb des Verfahrensschrittes d) ein Einschlagen der ersten Schnittkanten A des rechteckigen Materialabschnitts zur Ausbildung von zwei Stoßkanten, wobei insbesondere der Verfahrensschritt d) vor dem Verfahrensschritt c) erfolgt. Weiterhin kann als zusätzlicher Verfahrensschritte e) weiteres Einschlagen von Faltkanten zur Ausbildung weiterer Falt- oder Stoßkanten erfolgen.

Weiterhin bevorzugt werden in dem Verfahrensschritt b) aus den Schnittkanten B keine Stoßkanten gebildet. Es kann jedoch auch vorgesehen sein, dass im Verfahrensschritt b) zwei Faltkanten und zwei von diesen Faltkanten verschiedene Stoßkanten gebildet werden, wobei die Stoßkanten aus den Schnittkanten B gebildet werden. Anzumerken ist an dieser Stelle, dass erfindungsgemäße Kompressen sowohl maschinell als auch von Hand zu fertigen sind.

Mit einem erfindungsgemäßen Verfahren soll insbesondere ein Verfahren zur Herstellung einer medizinischen Kompresse bereitgestellt werden, die mindestens 8 und höchstens 16 Lagen eines Flachbahnmaterials umfasst. Ganz besonders bevorzugt handelt es sich bei dem erfindungsgemäßen Verfahren um ein Verfahren zur Herstellung einer Kompresse, die im Querschnitt betrachtet 8-lagige und 10- oder 12-lagige Bereiche aufweist. Hierunter ist zu verstehen, dass die Kompresse im Querschnitt betrachtet nicht über den gesamten Bereich ihrer Quer- oder Längserstreckung einen homogenen Lagenaufbau aufweist, sondern dass die Kompresse in einem ersten Teilbereich 10- oder 12-lagig und in mindestens einem weiteren Teilbereich 8-lagig ist. Es kann jedoch auch vorgesehen sein, das die Kompresse einen homogenen Lagenaufbau von entweder 10 Lagen oder 12 Lagen oder 16 Lagen aufweist.

Insbesondere soll ein Verfahren zur Herstellung einer Kompresse mit quadratischen Auflageflächen F mit dem Flächenmaß d² bereitgestellt werden, wobei d die Kantenlänge einer Randkante D der Kompresse ist. In diesem Verfahren wird bevorzugt als Ausgangsmaterial ein rechteckiger Materialabschnitt mit den Schnittkanten A und B verwendet, wobei dieser Materialabschnitt weiterhin bevorzugt eine Kantenlänge a mit a = 4 d + 2 e der Schnittkanten A und eine Kantenlänge b mit b = d + 2 e' der Schnittkanten B aufweist, wobei d die Kantenlänge der endgefalteten Kompresse, e die Kantenlänge eines Teilabschnitts der Schnittkante A mit e ≤ ½ d und e' die Kantenlänge eines Teilabschnitts der Schnittkante B mit e' ≤ ½ d ist. Auf diese Weise kann ohne großen Verschnitt oder Abfall lediglich durch präzise Faltung eine mindestens 8-lagige Kompresse und höchstens 10-lagige Kompresse bereitgestellt werden, die eine besonders gleichmäßige Materialverteilung über die Auflagefläche verteilt vorsieht.

Wird im Gegensatz hierzu der gleiche Materialabschnitt verwendet und die im Verfahrensschritt b) hergestellten Faltkanten nicht als Stoßkanten ausgebildet, so erhält eine auf 8 Lagen begrenzte Kompresse eine kleinere Auflagefläche. Dieser Sachverhalt anders ausgedrückt bedeutet, dass zur Herstellung einer Kompresse mit definierter Auflagefläche ein größerer Materialabschnitt bereitgestellt werden muss.

Besonders bevorzugt wird damit in diesem Verfahren der Verfahrensschritt b) vor dem Verfahrensschritt d) durchgeführt. Insbesondere werden in diesem Verfahren im Verfahrensschritt b) die Schnittkanten B des bereitgestellten Materialabschnitts eingeschlagen, die eine Kantenlänge b mit b = d + 2 e' aufweisen. Hierdurch wird im Gegensatz zu einem Verfahren in dem unter b) die Schnittkanten A eingeschlagen werden, die eine Kantenlänge von a = 4 d + 2 e aufweisen, eine Kompresse erhalten, die eine größere Auflagefläche bei gleichem Materialeinsatz aufweist.

Hervorzuheben ist an dieser Stelle, dass die hier aufgeführten Merkmale der bevorzugten oder alternativen Ausgestaltungen der Erfindungen nicht auf die einzelnen Bevorzugungen oder Alternativen zu beschränken sind. Es ist vielmehr der Fall, dass die Kombination der Ausgestaltungen bzw. die Kombination der Einzelmerkmale der alternativen Formen ebenso zu einer erfindungsgemäßen Ausgestaltung zu zählen ist. Ebenso wenig soll die Erfindung durch die nachfolgende Beschreibung der Zeichnungen reduziert verstanden sein.

In den Zeichnungen zeigen:
Figur 1: Ein Materialabschnitt zur Herstellung einer erfindungsgemäßen Kompresse in Aufsicht
Figur 2a: Zwischenprodukt zur Herstellung einer erfindungsgemäßen Kompresse in Aufsicht
Figuren 2b, 2c, 2d: Zwischenprodukt gemäß Figur 2a in verschiedenen Querschnitten
Figur 3a: Zwischenprodukt zur Herstellung einer erfindungsgemäßen Kompresse in Aufsicht
Figuren 3b, 3c, 3d: Zwischenprodukt gemäß Figur 3a in verschiedenen Querschnitten
Figur 4a: Eine erfindungsgemäße Kompresse in Aufsicht
Figuren 4b, 4c, 4d, 4e: Die Kompresse gemäß Figur 4a in verschiedenen Querschnitten
Figuren 5: Eine weitere erfindungsgemäße Kompresse in Aufsicht

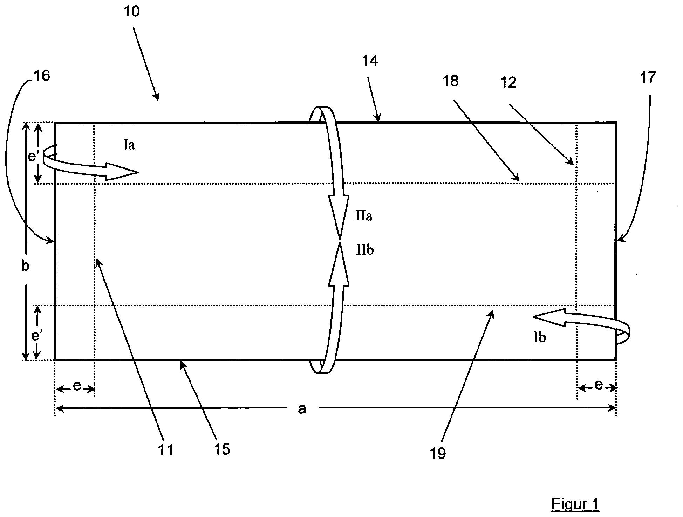

Mit Figur 1 ist ein rechteckiger Materialabschnitt (10) aus Mull gemäß DIN EN 14079 zur Herstellung einer erfindungsgemäßen Kompresse mit quadratischen Auflageflächen gezeigt. Dieser Materialabschnitt weist zwei sich gegenüberliegende erste Schnittkanten A (14, 15) mit der Kantenlänge a = 230,0 mm auf. Der Materialabschnitt weist weiterhin zwei sich gegenüberliegende zweite Schnittkanten B (16, 17) mit der Kantenlänge b = 99,0 mm auf.

Im Folgenden soll ein Verfahren zur Herstellung einer mindestens 8-lagigen Kompresse an Hand der Zeichnungen erläutert werden. Hierbei wird in einem ersten Verfahrensschritt der beschriebene rechteckige Materialabschnitt (10) bereitgestellt. In einem zweiten Verfahrensschritt werden die Schnittkanten B (16, 17) in Richtung der Pfeile Ia und Ib entlang der Faltlinien I (11, 12) zur Bildung von ersten Faltenkanten G (26) und G' (27) auf eine Oberseite des Materialabschnittes umgeschlagen. Durch das Umschlagen einer Schnittkante B (16, 17) werden zwei Untereinheiten des Materialabschnitts aufeinander gelegt, so dass die umgeschlagene Untereinheit als auch die verbleibende Untereinheit jeweils eine separate Lage bildet, wobei die gebildeten Lagen direkt benachbart und durch die gebildeten Faltkanten G (26) oder G' (27) verbunden sind. Die Abstände e der Faltlinien I zu der jeweils näher liegenden, parallelen Schnittkante B betragen jeweils e = 14,5 mm. In einem dritten Verfahrenschritt werden sodann die Schnittkanten A (14, 15) in Richtung der Pfeile IIa und IIb entlang der Faltlinien II (18, 19) zur Bildung von zweiten Faltkanten H (24) und H' (25) auf eine Oberseite des Materialabschnitts umgeschlagen. Die Abstände e' der Faltlinien II (18, 19) zu der jeweils näher liegenden, parallelen Schnittkante A betragen jeweils 24,5 mm. Durch das Umschlagen der Schnittkanten A (14, 15), werden die zweiten Faltkanten H (24) und H' (25) sowie aus Teilbereichen der Schnittkanten A gebildete Stoßkanten (28a, 29a; 28b, 29b, 28b', 29b') gebildet. Weitere Teilbereiche dieser Stoßkanten bilden die zweite Stoßkante der endgefertigten Kompresse.

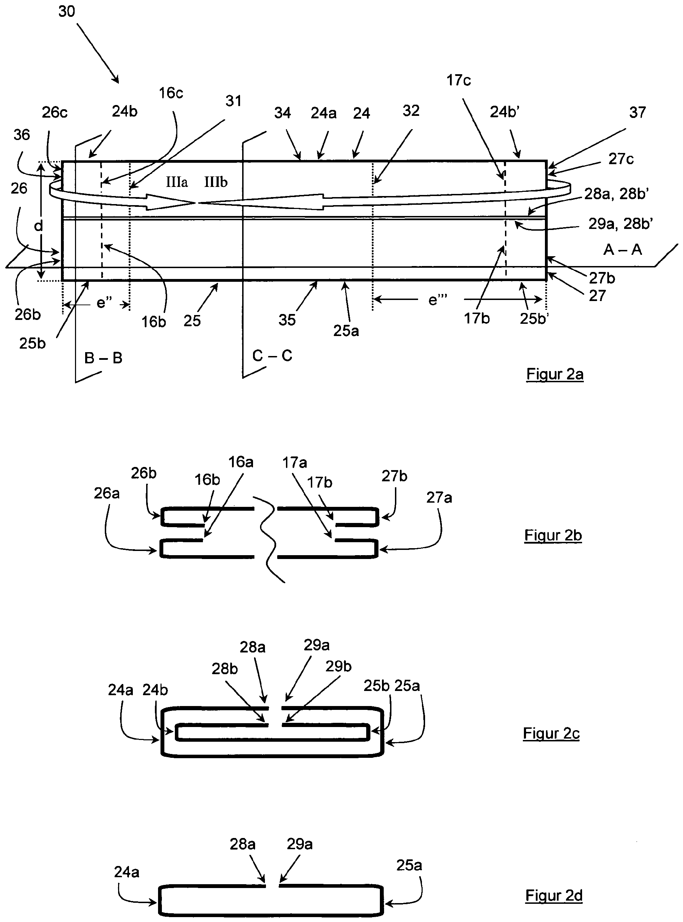

Mit Figur 2a ist das nach dem dritten Verfahrensschritt erhaltene Zwischenprodukt A (30) mit den zu den Schnittkanten B parallel liegenden Randkanten bb (36) und bb' (37) und den zu den Schnittkanten A parallel liegenden Randkanten aa (34) und aa' (35) aufgezeigt. Hierbei werden die Randkante aa (34) aus einem Teilabschnitt der zweiten Faltkante H (24a) und die Randkante aa' (35) aus einem Teilabschnitte der zweiten Faltkante H' (25a) gebildet (vgl. auch Figur 2c - Querschnitt des Zwischenproduktes A entlang der Schnittlinie B - B; sowie Figur 2d - Querschnitt des Zwischenproduktes A entlang der Schnittlinie C - C). Die von den die Randkanten bildenden Teilabschnitten eingeschlossenen weiteren Teilabschnitte der Faltkanten H und H' (24b, 25b, 24b', 25b') bilden keine Randkanten des Zwischenproduktes. Die Randkante bb (36) wird aus übereinander liegenden Teilabschnitten der ersten Faltkante G (26a, 26b, 26c) und die Randkante bb' (37) aus übereinander liegenden Teilabschnitten der ersten Faltkante G' (27a, 27b, 27c) gebildet (vgl. auch Figur 2b - Querschnitt des Zwischenproduktes A entlang der Schnittlinie A - A). Durch das Umschlagen der Schnittkanten A (14, 15) sind aus den Schnittkanten A Stoßkanten gebildet, wobei diese Stoßkanten jeweils 1,0 mm beabstandet sind. Alle Teilbereiche der Schnittkanten B (16a, 16b, 16c, 17a, 17b, 17c) sind durch darüber liegende Materialbereiche verdeckt (in den Figuren sind durch darüber liegende Materiallagen verdeckten Kanten gestrichelt dargestellt).

In einem weiteren Verfahrensschritt wird das Zwischenprodukt A (30) weiterverarbeitet. Hierzu werden die gebildeten Randkanten bb (36) und bb' (37) des Zwischenproduktes A in Richtung der Pfeile IIIa und IIIb entlang der Faltlinien III (31, 32) zur Bildung der ersten Stoßkanten (38a, 39a) auf eine Oberseite des Materialabschnittes umgeschlagen. Der Abstand e" der ersten Faltlinie III (31) von der nächstliegenden, parallelen Randkante aa (36) beträgt e" = 25,0 mm. Der Abstand e'" der zweiten Faltlinie III (32) von der nächstliegenden, parallelen Randkante aa' (37) beträgt e'" = 75,0 mm. Durch das Umschlagen der Randkanten bb (36) und bb' (37) werden weitere Faltkante I (41), I' (42), I" (43) und I'" (44) sowie die ersten Stoßkanten (38a, 39a) und weitere Stoßkanten (38b, 39b, 38c, 39c) einer weiter unten angeordneten Ebene der endgefertigten Kompresse gebildet. Hierbei ist jede gebildeten Stoßkante (38a, 39a, 38b, 39b, 38c, 39c) aus Teilabschnitten der Faltkanten G (26) und G' (27) gebildet, so dass die Stoßkanten direkt benachbarte Lagen verbinden. Es werden keine weiteren Lagen zwischen die verbundenen Lagen ein- oder zwischengeschoben.

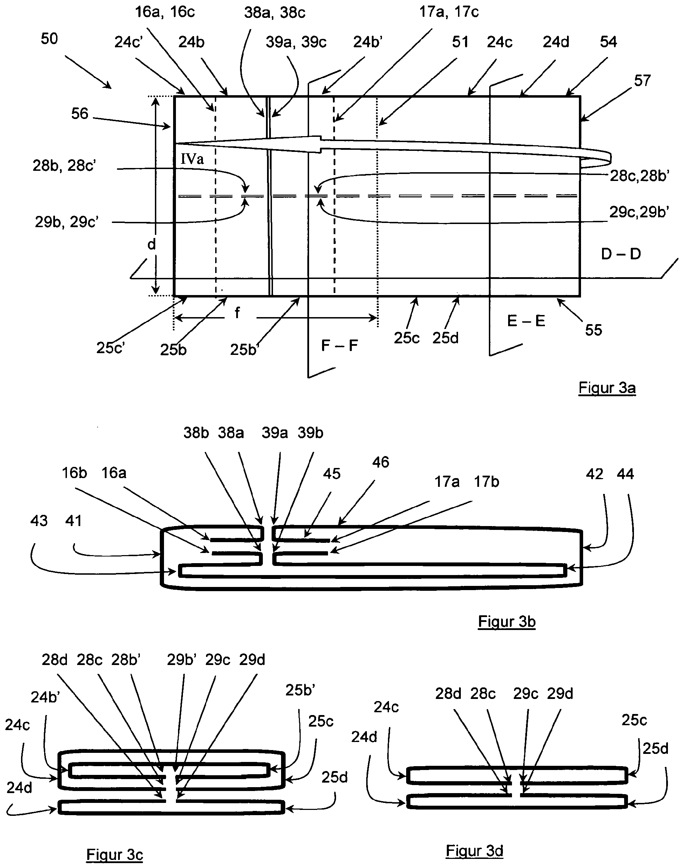

Mit Figur 3a ist das nach dem vierten Verfahrensschritt erhaltene Zwischenprodukt B (50) mit den zu den Schnittkanten B parallelen Randkanten bbb (56) und bbb' (57) sowie den zu den Schnittkanten A parallelen Randkanten aaa (54) und aaa' (55) wiedergegeben. Hierbei wird die Randkante aaa (54) durch übereinander liegende Teilabschnitte (24c, 24d, 24c') der zweiten Faltkante H (24) und analog die Randkante aaa' (35) durch übereinander liegende Teilabschnitte (25c, 25d, 25c') der zweiten Faltkante H' (25) gebildet (vgl. auch Figur 3c - Querschnitt des Zwischenproduktes B entlang der Schnittlinie F - F; sowie Figur 3d - Querschnitt des Zwischenproduktes A entlang der Schnittlinie E - E). Die von den Teilabschnitten eingeschlossenen weiteren Teilabschnitte der Faltkanten H und H' (24b, 25b, 24b', 25b') bilden keine Randkanten des Zwischenproduktes B. Die Randkante bbb (56) wird durch die weitere Faltkante I (41) und analog die Randkante aaa' (57) durch die weitere Faltkante I' (42) gebildet (vgl. Figur 3b - Querschnitt des Zwischenproduktes B entlang der Schnittlinie D - D). Die weiteren Faltkanten der inneren Lagen I" (43) und I'" (44) bilden keine Randkanten des Zwischenproduktes B (50). Die ersten Stoßkanten werden durch Teilabschnitte der Faltkanten G und G' (38a, 39a) gebildet. In einer weiteren Ebene werden aus weiteren Teilabschnitten der Faltkanten G und G' weitere Stoßkanten (38b, 39b, 38c, 39c) ausgebildet. Die ersten Stoßkanten (38a, 39a) liegen direkt aneinander, wobei ihr Abstand 1,0 mm beträgt. Der Abstand entspricht einem Abstand von 2 % bezogen auf die Kantenlänge d einer Randkante D der fertigen Kompresse. Sowohl die Schnittkanten B bzw. deren Teilabschnitte (16a, 16b, 17a, 17b, 16c, 17c) als auch die aus Teilabschnitten der Schnittkanten A gebildeten Stoßkanten (28b, 29b, 28b', 29b', 28c, 29c, 28c', 29c', 28d, 29d) werden durch darüber liegende Materiallagen verdeckt. Somit liegen in diesem Zwischenprodukt B (50) keine freien Schnittkanten vor.

In einem letzten Verfahrensschritt wird das Zwischenprodukt B (50) weiterverarbeitet. Hierbei wird die zuvor aus der Faltkante I' (42) gebildete Randkante bbb' (57) des Zwischenproduktes B (50) in Richtung des Pfeils IVa entlang der Faltlinie IV (51) umgeschlagen, so dass die umgeschlagene Randkante bbb' (57) auf der aus der Faltkante I gebildeten Randkante bbb (56) des Zwischenproduktes B (50) aufgelegt wird. Der Abstand der Faltlinie IV (51) zu der Randkante bbb (56) entspricht f = 50,5 mm. Durch diesen Verfahrensschritt wird entlang der Faltlinie IV (51) eine die erste Randkante D' (67) des fertigen Produktes bildende Faltkante J (47) sowie weitere Faltkanten J', J" und J'" (48a, 48b und 48c) gebildet.

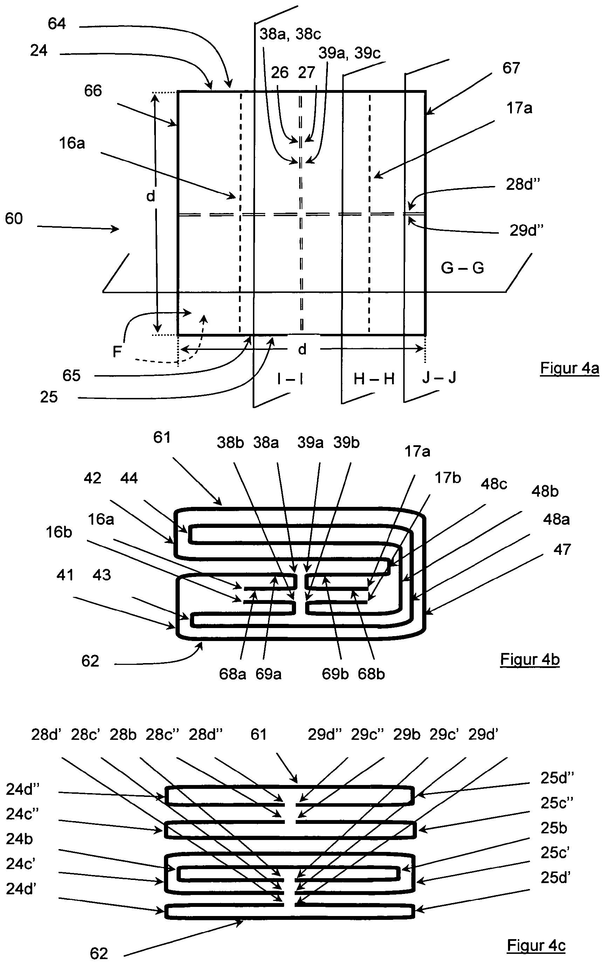

Mit Figur 4a ist die im zuvor beschriebenen Verfahren hergestellte Kompresse (60) abgebildet. Die Kompresse mit den vier gleichlangen Randkanten D', D", D'" und D"" (64, 65, 66 und 67) mit einer Kantenlänge d = 50,0 mm weist quadratische Auflageflächen F mit dem Flächenmaß d² = 25,0 cm², zwei als erste Stoßkanten ausgebildete Faltkanten (38a, 39a) und zwei Teilbereiche der Schnittkanten A als zweite Stoßkanten (28d", 29d") auf. Alle Teilbereiche der Schnittkanten B und alle Teilbereiche der Schnittkanten A sind durch weitere Materiallagen verdeckt. In Figur 4a sind nur die oben liegenden Teilbereiche der Schnittkante B (16a, 17a) und die die zweiten Stoßkanten bildenden Teilbereiche der Schnittkante A (28d", 29d") wiedergegeben. Die aus einem Teilbereich der Faltkante G (26) und Teilbereich der Faltkante G' (27) gebildeten ersten Stoßkanten (38a, 39a) liegen direkt aneinander, wobei ihr Abstand 1,0 mm beträgt. Der Abstand entspricht einem Abstand von 2 % bezogen auf die Kantenlänge d der Randkante D' (alle Kanten haben die gleiche Länge) der fertigen Kompresse. Die zweiten Stoßkanten (28d", 29d") sind 1,0 mm beabstandet, wobei der Abstand 2 % bezogen auf die Kantenlänge d einer Randkante D' entspricht. Die ersten Stoßkanten (38a, 39a) liegen in etwa in der Mitte der Kompresse und weisen in jedem Punkt einen Abstand zu der parallel liegenden ersten Randkante D' (67) von 49 % bzw. 51 % des Betrags der Länge einer zweiten, senkrecht zur ersten Randkante liegenden Randkante (64) auf. Des Weiteren liegen die ersten Stoßkanten (38a, 39a) senkrecht zu den zweiten Stoßkanten (28d", 29d").

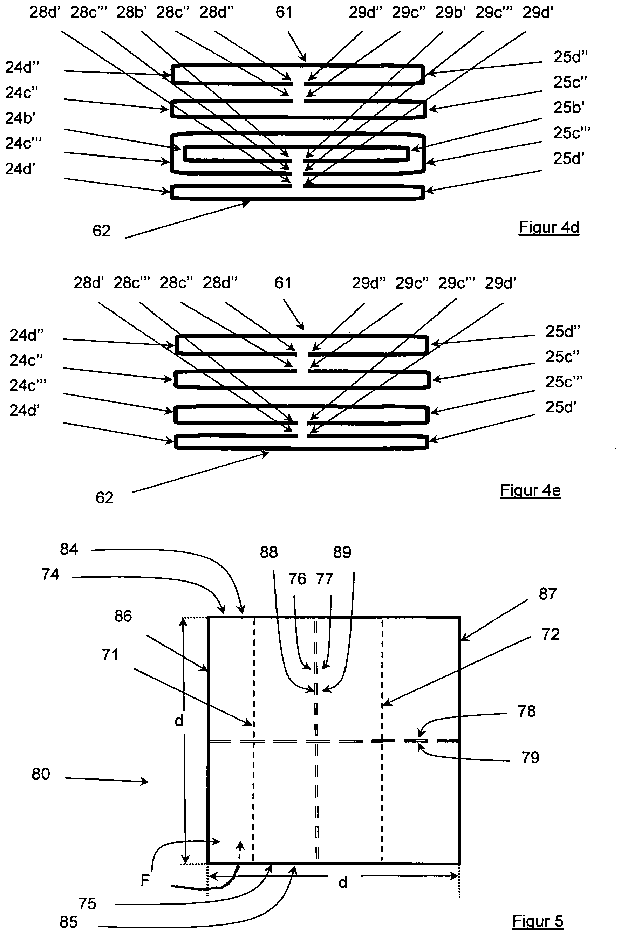

Mit den Folgenden Figur 4b, 4c, 4d und 4e soll die Lagigkeit der Kompresse (60) verdeutlicht werden, wobei mit Figur 4b der Querschnitt gemäß Schnittlinie G - G, mit Figur 4c der Querschnitt gemäß Schnittlinie I - I, mit Figur 4d der Querschnitt gemäß Schnittlinie H - H und mit Figur 4e der Querschnitt gemäß Schnittlinie J - J gezeigt ist. Zur Verdeutlichung der Lagigkeit der Kompresse (60) sind die einzelnen Lagen sowie ineinander liegende Faltkanten - wie in allen Querschnittszeichnungen - auseinander gezogen bzw. voneinander beabstandet dargestellt. Insbesondere ist gezeigt, dass die beiden äußeren die Auflageflächen F bildenden Lagen (61, 62) durch die Faltkante J (47), die die erste Randkante D' (67) der Kompresse bildet, miteinander verbunden sind. Jede weitere Lage ist von diesen beiden äußeren Lagen ummantelt, so dass alle weiteren Lagen zwischen den äußeren Lagen (61, 62) liegen.

Des Weiteren ist gezeigt, dass die zweite Randkante D" (64) der Kompresse aus übereinander liegenden Teilabschnitten der Faltkante H (24c"', 24d', 24c", 24d", 24c'), die dritte Randkante D'" (45) aus übereinander liegenden Teilabschnitten der Faltkante H' (25c'", 25d', 25c", 25d", 25c') und die vierte Randkante D"" (66) aus den übereinander liegenden Faltkanten I (41) und I' (42) gebildet ist. Die jeweils innen liegenden Faltkanten (43, 44, 48a, 48b, 48c) sowie die weiteren Teilabschnitte der Faltkanten H (24b, 24b') und H' (25b, 25b') bilden keine Randkanten der Kompresse. Damit weist die Kompresse lediglich solche Randkanten auf, die durch Faltkanten gebildet werden. Weiterhin weist die Kompresse deckungsgleiche erste Stoßkanten (38a, 39a) und aus Faltkanten gebildete weitere Stoßkanten (38b, 39b) auf. Diese Stoßkanten sind aus den Faltkanten G (26) und G' (27) gebildet. Jede dieser ersten Stoßkanten (38a, 39a) verbindet eine erste Lage direkt mit einer zweiten Lage, wobei die erste Lage aus einem ersten Teilabschnitt (68a) und einem zweiten Teilabschnitt (68b) sowie die zweite Lage aus einem ersten Teilabschnitt (69a) und einem zweiten Teilabschnitt (69b) gebildet sind (vgl. Figur 4b). Senkrecht hierzu liegen die deckungsgleichen, aus den Schnittkanten A gebildeten zweiten Stoßkanten (28d", 29d") und weitere aus den Schnittkanten A gebildete Stoßkanten (28c'", 29c'"; 28c", 29c", 28c', 29c'; 28d', 29d'; 28b, 29b, 28b', 29b').

Jede Lage dieser Kompresse ist mittels mindestens einer Faltkante mit einer weiteren Lage der Kompresse verbunden, wobei die Kompresse im Querschnitt betrachtet 8-lagige und 10-lagige Bereiche aufweist. Die Kompresse weist als 8-lagige Kompresse einen mittleren Teilbereich auf, der 10-lagig ist. Die beiden zusätzlichen Lagen werden durch das Umschlagen von den Schnittkanten B (16, 17) erhalten (vgl. Figur 4b). In den Randbereichen weist die Kompresse 8 Lagen auf (vgl. Figur 4d). Diese Kompresse weist als 8-lagige Kompresse auch nach einmaligem Entfalten keine freien Schnittkanten auf (vgl. Figur 3a). Somit könnte diese Kompresse als 4-lagige oder 8-lagige Kompresse verwendet werden. Die Kompresse (60) weist im mittleren Bereich (10 Lagen) eine Dicke von 1,24 mm und im Randbereich (8 Lagen) eine Dicke vom 1,12 mm auf, jeweils gemessen bei einem Prüfdruck von 2 g/cm² (vgl. unten). Damit kann eine Mehrzahl dieser Kompressen leicht gestapelt werden, da sie über alle Bereiche eine gleichmäßige Materialverteilung aufweist.

Im Folgenden soll ein Vergleich der Stapelhöhen angegeben werden. Werden beispielsweise im Markt befindliche ES-Kompressen (ES-Kompressen 5x5 cm - Paul Hartmann AG) gestapelt, so weist die Verpackung mit einem Kompressenstapel von 100 Kompressen eine Höhe von 155 mm (Außenmaß der Verpackung) auf. Werden dagegen 100 Stück erfindungsgemäße Kompressen (60) gestapelt und unter denselben Bedingungen mit demselben Verpackungsmaterial verpackt, so ist ein Außenmaß von 130 mm zu verzeichnen (dieselben Messbedingungen). Das entspricht einer Reduzierung in der Stapelhöhe von ca. 16 %.

Für einen Teilstapel von 5 Kompressen bzw. jede einzelne Kompresse ergeben sich folgende Werte, wobei ein Prüfdruck 2g/ cm² eingehalten wurde. Jede Kompresse ist identisch gefaltet, wobei die Kompressen im Stapel deckungsgleich gestapelt sind.

| | | Dicke (5 Stück) / mm | Dicke (1 Stück) / mm |
|---|---|---|---|
| ES-Kompresse (Paul Hartmann AG) | Randkante (16 Lagen) | 8,10 | 1,62 |
| | parallele Gegenkante (8 Lagen) | 5,65 | 1,13 |
| Erfindungsgemäße Kompresse (60) | Randbereich (8 Lagen) | 5,60 | 1,12 |
| | mittlerer Bereich (10 Lagen) | 6,20 | 1,24 |

Durch die Anordnung der Faltkanten als Stoßkanten kann ein Kompressenstapel bereitgestellt werden, der im Vergleich zu im Markt befindlichen Kompressen in sich stabiler ist und weniger Platz einnimmt.

In der folgenden Tabelle sind Beispiele quadratischer Kompressen angegeben, die gemäß des oben beschriebenen Verfahrens aus einem rechteckigen Materialabschnitt mit zwei sich gegenüber liegenden parallelen Schnittkanten A und zwei sich gegenüber liegenden parallelen Schnittkanten B gebildet sind. Die Kantenlänge a der Schnittkanten A ist größer als die Kantenlänge b der Schnittkanten B des bereitgestellten Materialabschnitts. Die verglichenen Kompressen weisen im endgefalteten Zustand die jeweils angegebene Randkantenlänge d auf. Anhand der Tabelle soll verdeutlicht werden, inwieweit bei gleichem Material eine Materialersparnis gegenüber bekannten ES-Kompressen - Paul Hartmann AG (1. Faltung) verwirklicht ist, wobei bei den zum Vergleich herangezogenen ES-Kompressen die ersten Faltkanten parallel zu den Schnittkanten A gebildet sind.

| Fläche des eingesetzten Materialabschnitts F_{M} = a · b | Kompresse 1 d = 5 cm | | | Kompresse 2 d = 7,5 cm | Kompresse 3 d = 10 cm |
|---|---|---|---|---|---|
| | e = 1,0 cm | e = 1,5 cm | e = 2,0 cm | e = 1,5 cm | e = 1,5 cm |
| 1. Faltung ES-Kompresse F_{M} = 4d · (2d + 2e) | 240 cm² | 260 cm² | 280 cm² | 540 cm² | 920 cm² |
| 2. Faltung (erfindungsgemäß) F_{M} = (4d + 2e) · 2d | 220 cm² | 230 cm² | 240 cm² | 495 cm² | 860 cm² |
| Materialersparnis | 20 cm² (9,1%) | 30 cm² (11,5%) | 40 cm² (14,3%) | 45 cm² (8,3%) | 60 cm² (6,5%) |

So beläuft sich beispielsweise die Materialersparnis für eine erfindungsgemäße Kompresse mit quadratischen Auflageflächen und einer Kantenlänge d mit d = 5 cm (Kompresse 1) bei gleicher Breite e des im ersten Schritt umgeschlagenen Abschnittes mit e = 1,5 cm auf ca. 11,5% gegenüber etablierten ES-Kompressen. Hierbei weisen sowohl die ersten als auch die zweiten Stoßkanten einen Abstand von 1,0 mm auf. Die Materialersparnis kann somit durch präzise Faltung in neuartiger Art erzielt werden, wobei durch die Faltung die erfindungsgemäßen Stoßkanten verwirklicht sind.

Mit Figur 5 ist ein weiteres Beispiel einer 8-lagige Mullkompresse wiedergegeben, die quadratische Auflageflächen aufweist, Die Kantenlänge d der Randkante D der Kompresse (80) beträgt d = 75,0 mm. Die Kompresse ist gemäß dem zuvor beschriebenen Verfahren hergestellt worden, wobei folgende Maße eingehalten wurden: a = 330,0 mm, b = 149,0 mm, e = 14,5 mm, e' = 37,0 mm, e" = 30,0 mm, e'" = 120,0 mm und f = 75,5 mm. Damit weist diese Kompresse vier gleichlange Randkanten D', D", D"', D"" (84, 85, 86, und 87), verdeckte Schnittkanten B (71, 72), als erste Stoßkanten ausgebildeten Faltkanten (88, 89) sowie als zweite Stoßkanten ausgebildeten Schnittkanten A (78, 79) auf. Der Abstand der gebildeten zweiten Stoßkanten (78, 79) beträgt 1,0 mm voneinander (1,3 % bezogen auf die Kantenlänge d der Randkante D' (87) der Kompresse). Der Abstand der gebildeten ersten Stoßkanten (88, 89) beträgt 1,0 mm voneinander (1,3 % bezogen auf die Kantenlänge d der Randkante D' (87) der Kompresse). Der Abstand der ersten Stoßkanten (88, 89) von der ersten parallel zu den Stoßkanten liegenden Randkante D' (87) beträgt 60 % bzw. 58,6 % bezogen auf die Kantenlänge einer zu der ersten senkrecht stehenden zweiten Randkante D" (84) der Kompresse. Wird der Abstand auf die zweite, parallel zu den ersten Stoßkanten liegende Randkante D'" (86) bezogen, so beträgt der Abstand der 40 % bzw. 41,3 %. In jedem Fall liegen die Stoßkanten in einem mittleren Bereich der Auflageflächen der Kompressen, der sich parallel zu einer ersten Rankante in Richtung der zweiten parallelen Randkante im Abstand von 25 bis 75 % der Kantenlänge derjenigen Kante erstreckt, die sich ihrerseits senkrecht zu der ersten oder zweiten Randkante erstreckt.

Diese hier gezeigten medizinischen Kompressen können insbesondere in der Notfallmedizin als auch bei operativen Eingriffen verwendet werden. Sie zeichnen sich durch eine besondere Sicherheit im Gebrauch und durch eine besonders gleichmäßige Materialverteilung aus.

## Patentansprüche

1. Medizinische Kompresse (60, 80) umfassend mindestens 8 Lagen eines textilen Flachbahnmaterials, wobei jede Lage über mindestens eine Faltkante mit einer weiteren Lage verbunden ist und mindestens eine erste Faltkante (26, 27, 76, 77) und eine zweite Faltkante (24, 25, 74, 75) senkrecht zueinander stehen, **dadurch gekennzeichnet, dass** die Kompresse derart gefaltet ist, dass die Kompresse mindestens zwei Faltkanten als erste Stoßkanten (38a, 39a, 88, 89) umfasst, wobei jede dieser ersten Stoßkanten (38a, 39a, 88, 89) direkt benachbarte Lagen oder Teilabschnitte der direkt benachbarten Lagen verbindet und dass jede Randkante (64, 65, 66, 67, 84, 85, 86, 87) der Kompresse ausschließlich aus Faltkanten gebildet ist, wobei die Kompresse derart gefaltet ist, dass die äußeren, die Auflageflächen der Kompresse bildenden Lagen die Form eines Rechtecks oder eines Quadrates aufweisen.

2. Medizinische Kompresse nach Anspruch 1, **dadurch gekennzeichnet, dass** die Kompresse derart gefaltet ist, dass die äußeren, die Auflageflächen der Kompresse bildenden Lagen (61, 62) vollständig durch jeweils einen zusammenhängenden Bereich des Flachbahnmaterials gebildet sind.

3. Medizinische Kompresse nach mindestens einem der vorgenannten Ansprüche, **dadurch gekennzeichnet, dass** die ersten Stoßkanten (38a, 39a, 88, 89) in jedem Punkt einen Abstand zu einer parallelen ersten Randkante (66, 67, 86, 87) von mindestens 25 % und höchstens 75 % des Betrags der Länge einer zweiten, senkrecht zur ersten Randkante liegenden Randkante (64, 65, 84, 85) aufweisen.

4. Medizinische Kompresse nach mindestens einem der vorgenannten Ansprüche, **dadurch gekennzeichnet, dass** die Kompresse zwei weitere Schnitt- oder Faltkanten als zweite Stoßkanten (28d", 29d", 78, 79) umfasst.

5. Medizinische Kompresse nach mindestens einem der vorgenannten Ansprüche, **dadurch gekennzeichnet, dass** die ersten Stoßkanten (38a, 39a, 88, 89) senkrecht zu den zweiten Stoßkanten (28d", 29d", 78, 79) liegen.

6. Medizinische Kompresse nach mindestens einem der vorgenannten Ansprüche, **dadurch gekennzeichnet, dass** die Kompresse im Querschnitt betrachtet 8-lagige und 10-lagige Bereiche aufweist.

7. Medizinische Kompresse nach mindestens einem der vorgenannten Ansprüche, **dadurch gekennzeichnet, dass** die Kompresse 10 Lagen aufweist.

8. Kompressenstapel umfassend eine Mehrzahl von Kompressen (60, 80) gemäß mindestens einem der vorgenannten Ansprüche.

## Claims

1. Medical compress (60, 80) comprising at least 8 layers of a flat web textile material, each layer being connected to a further layer via at least one folded edge, and at least one first folded edge (26, 27, 76, 77) and one second folded edge (24, 25, 74, 75) being perpendicular to each other, **characterized in that** the compress is folded in such a way that the compress comprises at least two folded edges as first abutment edges (38a, 39a, 88, 89), wherein each of these first abutment edges (38a, 39a, 88, 89) connects directly adjacent layers or partial sections of the directly adjacent layers, and **in that** each outer edge (64, 65, 66, 67, 84, 85, 86, 87) of the compress is formed exclusively of folded edges, the compress being folded in such a way that the outer layers forming the contact surfaces of the compress have the shape of a rectangle or of a square.

2. Medical compress according to Claim 1, **characterized in that** the compress is folded in such a way that the outer layers (61, 62) forming the contact surfaces of the compress are formed completely by in each case a continuous region of the flat web material.

3. Medical compress according to at least one of the preceding claims, **characterized in that** the first abutment edges (38a, 39a, 88, 89) are at each point at a distance from a parallel first outer edge (66, 67, 86, 87) of at least 25% and at most 75% of the length of a second outer edge (64, 65, 84, 85) lying perpendicular to the first outer edge.

4. Medical compress according to at least one of the preceding claims, **characterized in that** the compress comprises two further cut or folded edges as second abutment edges (28d", 29d", 78, 79).

5. Medical compress according to at least one of the preceding claims, **characterized in that** the first abutment edges (38a, 39a, 88, 89) lie perpendicular to the second abutment edges (28d", 29d", 78, 79).

6. Medical compress according to at least one of the preceding claims, **characterized in that** the compress, viewed in cross section, has 8-layer and 10-layer regions.

7. Medical compress according to at least one of the preceding claims, **characterized in that** the compress has 10 layers.

8. Stack of compresses, comprising a plurality of compresses (60, 80) according to at least one of the preceding claims.

## Revendications

1. Compresse médicale (60, 80) comprenant au moins 8 couches d'un matériau en bande plate textile, chaque couche étant connectée à une couche supplémentaire par le biais d'au moins une arête de pliage et au moins une première arête de pliage (26, 27, 76, 77) et une deuxième arête de pliage (24, 25, 74, 75) étant perpendiculaires l'une à l'autre, **caractérisée en ce que** la compresse est pliée de telle sorte que la compresse comprenne au moins deux arêtes de pliage en tant que premières arêtes de butée (38a, 39a, 88, 89), chacune de ces premières arêtes de butée (38a, 39a, 88, 89) reliant des couches directement adjacentes ou des portions partielles des couches directement adjacentes et **en ce que** chaque arête de bord (64, 65, 66, 67, 84, 85, 86, 87) de la compresse est formée exclusivement d'arêtes de pliage, la compresse étant pliée de telle sorte que les couches extérieures formant les surfaces d'appui de la compresse présentent la forme d'un rectangle ou d'un carré.

2. Compresse médicale selon la revendication 1, **caractérisée en ce que** la compresse est pliée de telle sorte que les couches extérieures (61, 62) formant les surfaces d'appui de la compresse soient formées complètement à chaque fois par une zone cohésive du matériau en bande plate.

3. Compresse médicale selon au moins l'une quelconque des revendications précédentes, **caractérisée en ce que** les premières arêtes de butée (38a, 39a, 88, 89) présentent en chaque point, une distance à une première arête de bord parallèle (66, 67, 86, 87) d'au moins 25 % et d'au plus 75 % de la valeur de la longueur d'une deuxième arête de bord (64, 65, 84, 85) située perpendiculairement à la première arête de bord.

4. Compresse médicale selon au moins l'une quelconque des revendications précédentes, **caractérisée en ce que** la compresse comprend deux arêtes de coupe ou de pliage supplémentaires en tant que deuxièmes arêtes de butée (28d", 29d", 78, 79).

5. Compresse médicale selon au moins l'une quelconque des revendications précédentes, **caractérisée en ce que** les premières arêtes de butée (38a, 39a, 88, 89) sont perpendiculaires aux deuxièmes arêtes de butée (28d", 29d", 78, 79).

6. Compresse médicale selon au moins l'une quelconque des revendications précédentes, **caractérisée en ce que** la compresse présente, vu en section transversale, des zones à 8 couches et à 10 couches.

7. Compresse médicale selon au moins l'une quelconque des revendications précédentes, **caractérisée en ce que** la compresse présente 10 couches.

8. Empilement de compresses comprenant une pluralité de compresses (60, 80) selon au moins l'une quelconque des revendications précédentes.
